(19) Europäisches Patentamt
European Patent Office
Office européen des brevets

(11) EP 1 939 304 A1

(12) **EUROPEAN PATENT APPLICATION**

(43) Date of publication:
**02.07.2008 Bulletin 2008/27**

(51) Int Cl.:
***C12Q 1/68*** *(2006.01)*

(21) Application number: **06077343.9**

(22) Date of filing: **29.12.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Nederlandse Organisatie voor Toegepast-Natuurwetenschappelijk Onderzoek TNO**
**2628 VK Delft (NL)**

(72) Inventors:
- **Schuren, Frank Henri Johan**
**3902 EB Veenendaal (NL)**
- **Montijn, Roy Christiaan**
**1028 BJ Amsterdam (NL)**
- **Thijssen, Henricus Matheus Wilhelmus M.**
**3994 TT Houten (NL)**

(74) Representative: **van Loon, C.J.J. et al**
**VEREENIGDE**
**Johan de Wittlaan 7**
**2517 JR Den Haag (NL)**

(54) **Method of detecting pathogenic Legionella strains**

(57) The invention relates to a method of detecting pathogenic *Legionella pneumophila* strains by hybridizing genomic DNA of a sample suspected to contain Legionella to two or five specific sequence markers, as identified by SEQ ID NO:1 through SEQ ID NO:5 or homologues thereof.. The invention further relates to a kit of parts comprising an array and reference materials for performing a method of the invention.

EP 1 939 304 A1

**Description**

FIELD OF THE INVENTION

The invention relates to a method of typing *Legionella pneumophila* strains, and in particular to a method of identifying a *Legionella pneumophila* strain as being either pathogenic or non-pathogenic. The invention further relates to markers for such an assay and a kit of parts comprising an array with said markers and reference materials for performing a method of the invention.

BACKGROUND OF THE INVENTION

Legionnaires' disease is an acute pneumonic illness caused by Gram-negative bacilli of the genus *Legionella*, the most common of which is *Legionella pneumophila*.

Legionnaires' disease is initiated by inhalation, and probably microaspiration, of *Legionella* bacteria into the lungs. Although *Legionella* bacteria are ubiquitous in our environment, they rarely cause disease. A number of factors must occur simultaneously before legionnaires' disease is possible. These factors include the presence of virulent strains in an environmental site; a means for dissemination of the bacteria, such as by aerosolization; and proper environmental conditions allowing the survival and inhalation of an infectious dose of the bacteria by a susceptible host.

Water contaminated with a sufficient concentration of virulent *Legionella* bacteria can be aerosolized by water-cooled heat rejection devices such as air conditioning cooling towers, whirlpool spas, shower heads, water misters, and the like. Once the bacteria enter the lung, they are phagocytosed by alveolar macrophages and then grow intracellularly. The *Legionella* bacteria produce virulence factors that enhance phagocytosis. After sufficient intracellular growth, the bacteria kill the macrophage, escape into the extracellular environment and are then rephagocytosed by other macrophages. Within a few days after initial infection, the bacterial concentration in the lung increases considerably. The resulting infiltration of the alveoli by neutrophils, additional macrophages and erythrocytes results in capillary leakage and edema and the chemokines and cytokines released by the macrophages help trigger a severe inflammatory response, which may be fatal.

More than 49 different *Legionella* species, encompassing 70 serogroups, have been described since its first discovery in 1977, 20 of which have been reported to infect humans. *L. pneumophila* contains at least 16 different serogroups. *L. pneumophila* serogroup 1 caused the 1976 Philadelphia outbreak and is the cause of 70% to 90% of all cases of legionnaires' disease. In the major outbreaks such as those originating in Bovenkarspel, The Netherlands in 1999, in Barrow-in-Furness in Cumbria, England in 2002 and near Harnes in Pas-de-Calais, France in 2003/2004, serogroup 1 strains could be detected in the majority of the patients.

*L. pneumophila* serogroup 1 can be further divided into multiple subtypes using a variety of serologic, other phenotypic and genetic methods. One particular subtype of *L. pneumophila* serogroup 1 causes the majority of cases of legionnaires' disease due to *L. pneumophila*, and 85% of the cases due to *L. pneumophila* serogroup 1; this subtype is distinguished by its reactivity with a particular monoclonal antibody, and it is variously termed Pontiac, the Joly monoclonal type 2 (MAb2), or the Dresden monoclonal type 3/1 (MAb 3/1) monoclonal subtype.

Most clinical microbiology laboratories are capable of identifying *Legionella* bacteria to the genus level by detection of their typical colony morphology, Gram stain appearance, and various other standard microbiology identification techniques. Identification of *L. pneumophila* serogroup 1, the most common clinical isolate can be accomplished by sophisticated clinical microbiology laboratories using relative simple serologic testing. However, it should be stressed that serogroup 1 strains are not the only virulent serotypes. Identification of other *L. pneumophila* serogroups, and other *Legionella* species, is often much more difficult. This is because these bacteria are relatively inert in the use of commonly tested biochemical substrates, and they require sophisticated phenotypic, serologic and molecular testing. Reference laboratory-based phenotypic testing of bacteria, including determination of fatty-acids and ubiquinones and protein electrophoresis, can often be used to identify the bacteria. Definitive identification is based on both immunologic detection of surface antigens and bacterial DNA sequencing. Also, DNA typing by pulsed field electrophoresis of DNA restriction fragments is useful for identification purposes.

Diagnosis of the infecting agent from clinical material (such as sputum or bronchoalveolar lavage fluid) on selective media is currently the most reliable means of diagnosis. However, cultivation is slow. Direct fluorescent antibody (DFA) stains for the visualization of *Legionella* species in clinical specimens are commercially available for a limited number of species. Assays for the detection of *L. pneumophila* serogroup 1 antigen in urine have a sensitivity of 70% and a specificity of nearly 100%. Also, an immunochromatographic assay for the rapid qualitative detection of *L. pneumophila* serogroup 1 antigen (Legionella NOW; Binax, Portland, Maine) in urine specimens has become available that uses rabbit anti-L. *pneumophila* serogroup 1 antibody as the capture component and rabbit anti-L. *pneumophila* serogroup 1 antibody conjugated to colloidal gold as the detection component. The assay provides a test result in 15 min and is intended to aid in the presumptive diagnosis of Legionnaires' disease caused by *L. pneuntophila* serogroup 1 in conjunction with culture and other methods. Preliminary performance data for the immunochromatographic assay report a sensitivity of 95% and a specificity of 95%. Thus, so far, commercially available tests for *Legionella* urinary antigen detect only *L. pneumophila* serogroup 1, while the specificity of the assays cannot prevent the occurrence of false positive and false negative reactions.

DNA probe techniques, which produce fewer false positive reactions then immunological detection methods, may be used to detect the presence of one or more multiple *Legionella* species. However, a drawback of such DNA methods is that they cannot differentiate between virulent and non-virulent strains, presumably because the virulence trait is multi-genic.

Following severe outbreaks, many national authorities have implemented legislation and water quality standards for water supplies and/or codes of practice for management and operation of cooling towers and warm water storage facilities. Such standards and codes require frequent monitoring of drinking water distribution systems and swimming-pool water facilities, and upon exceeding a certain number of *Legionella* bacteria per liter, rigorous measures are taken, such as closure and evacuation of hotels, sports facilities or nursing homes. Most experts however, consider that a large number of *Legionella* bacteria detected are harmless and non-virulent. However, there is at present no assay system available to distinguish between virulent and non-virulent strains and it is difficult to avoid the costs involved with false-alarm *Legionella* detection. The availability of a test that is capable of reliably detecting pathogenic *Legionella* strains would be highly favorable.

It is an object of the present invention to provide for a method capable of distinguishing between clinically relevant and environmental strains of *L. pneumophila*. It is a further object of the present invention to provide a method of detecting pathogenic *Legionella* strains including, but not limited to serogroup 1 strains.

## SUMMARY OF THE INVENTION

The present inventors have now found a method for distinguishing between clinical or pathogenic strains and environmental or non-pathogenic *Legionella* strains. The method involves an hybridization assay with specific genetic markers.

In one embodiment of the present invention, the group of molecular markers comprises the two markers indicated in SEQ ID NO:1 and SEQ ID NO:2 or sequences which are highly homologous to those.

In a preferred embodiment of the method of the present invention, the group of molecular markers comprises the five markers indicated in SEQ ID NO:1, SEQ ID NO:2, SEQ ID NO:3, SEQ ID NO:4 and SEQ ID NO:5, or homologues thereof.

In a further embodiment of the method of the present invention, the sample nucleic acid is derived from a pure culture of *Legionella pneumophila*. Alternatively, the sample can be derived from any medium which is suspected of *Legionella* infestation, such as aqueous samples from water pipes, surface water, drink water, showers, baths, fountains, etc.

A kit of parts, said kit comprising an array as defined herein together with reference nucleic acids as defined herein. Preferably, said kit also comprises software to calculate the result of the test.

## DETAILED DESCRIPTION OF THE INVENTION

The term *"Legionella pneumophila* strain" as used herein refers to the descendants of a single isolate of *Legionella pneuniophila* cells in pure culture and to a taxonomic level of said pure culture below the level of the species *Legionella pneuniophila*.

The term "pathogenic" in relation to a *Legionella* strain refers to a microorganism capable of causing disease (including infection) or morbid symptoms in humans or other animal hosts.

The term "array" refers to an array of individual fragments of DNA or oligonucleotides that are bound to a substrate such as a microscope slide. The purpose of an array experiment is to determine the amount of matching nucleic acid fragments in a particular sample. The target nucleic acid fragments are extracted from the cells or tissues of interest, optionally converted to DNA, and labeled. They are then hybridized to all the DNA or oligonucleotide spots on the array. Matches (i.e. the spots where hybridization has taken place) are identified using antibody detection, optionally combined with precipitation, chromatography, colorimetry, and/or phosphorescent or fluorescent imaging. The data resulting from an array experiment are a list of measurements of spot intensities. A preferred array of the invention is an array which has less than 1000, preferably less than 100, more preferably less than 10 spots, and which contains at least the DNA sequences of SEQ ID NO:1 and SEQ ID NO:2. Another preferred array of the invention is an array which has less than 1000, preferably less than 100, more preferably less than 10 spots, and which contains at least the five sequences as presented in SEQ ID NO:1 through SEQ ID NO:5.

The term "nucleic acid" as used herein, is interchangeable with the term "polynucleotide", and refers to a nucleotide multimer or polymeric form of nucleotides having any number of nucleotides, e.g., deoxyribonucleotides or ribonucleotides, or compounds produced synthetically (e.g. PNA as described in U.S. Pat. No. 5,948,902 and the references cited therein) and can be either double- or single-stranded. A polynucleotide can hybridize with other polynucleotides in a sequence specific manner, e.g. can participate in Watson-Crick base pairing interactions. The term also includes modified, for example by methylation and/or by capping, and unmodified forms of the polynucleotide

The terms "deoxyribonucleic acid" and "DNA" as used herein mean a polymer composed of deoxyribonucleotides.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double- and single-stranded DNA and double- and single-stranded RNA with circular, branched or linear shapes and optionally including domains capable of forming secondary structures (e.g., stem-loop, pseudo knots and kissing loop structures).

The term "nucleotide sequence homology" as used herein denotes the presence of homology between two polynucleotides. Polynucleotides have "homologous" sequences if the sequence of nucleotides in the two sequences is the same when aligned for maximum correspondence. Sequence comparison between two or more polynucleotides is generally performed by comparing portions of the two sequences over a comparison window to identify and compare local regions of sequence similarity. The comparison window is generally from about 20 to 200 contiguous nucleotides. The "percentage of sequence homology" for polynucleotides, such as 50, 60, 70, 80, 90, 95, 98, 99 or 100 percent sequence homology may be determined by comparing two optimally aligned sequences over a comparison window, wherein the portion of the polynucleotide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by visual inspection. Readily available sequence comparison and multiple sequence alignment algorithms are, respectively, the Basic Local Alignment Search Tool (BLAST) (Altschul et al., 1990; Altschul et al., 1997) and ClustalW programs, both available on the internet. Other suitable programs include, but are not limited to, GAP, BestFit, PlotSimilarity, and FASTA in the Wisconsin Genetics Software Package (Genetics Computer Group (GCG), Madison, WI, USA) (Devereux et al., 1984).

As used herein, "substantially complementary" means that two nucleic acid sequences have at least about 65%, preferably about 70%, more preferably about 80%, even more preferably 90%, and most preferably about 98%, sequence complementarity to each other. This means that primers and probes must exhibit sufficient complementarity to their template and target nucleic acid, respectively, to hybridise under stringent conditions. Therefore, the primer and probe sequences need not reflect the exact complementary sequence of the binding region on the template and degenerate primers can be used. For example, a non-complementary nucleotide fragment may be attached to the 5'-end of the primer, with the remainder of the primer sequence being complementary to the strand. Alternatively, non-complementary bases or longer sequences can be interspersed into the primer, provided that the primer has sufficient complementarity with the sequence of one of the strands to be amplified to hybridize therewith, and to thereby form a duplex structure which can be extended by the polymerising means. The non-complementary nucleotide sequences of the primers may include restriction enzyme sites. Appending a restriction enzyme site to the end(s) of the target sequence would be particularly helpful for cloning of the target sequence. A substantially complementary primer sequence is one that has sufficient sequence complementarity to the amplification template to result in primer binding and second-strand synthesis. The skilled person is familiar with the requirements of primers to have sufficient sequence complementarity to the amplification template.

The term "hybrid" in the context of nucleic acids refers to a doublestranded nucleic acid molecule, or duplex, formed by hydrogen bonding between complementary nucleotide bases. The terms "hybridise" or "anneal" refer to the process by which single strands of nucleic acid sequences form double-helical segments through hydrogen bonding between complementary bases.

The term "probe" refers to a single-stranded oligonucleotide sequence that will recognize and form a hydrogen-bonded duplex with a complementary sequence in a target nucleic acid sequence analyte or its cDNA derivative.

The term "primer" as used herein refers to an oligonucleotide which is capable of annealing to the amplification target allowing a DNA polymerase to attach, thereby serving as a point of initiation of DNA synthesis when placed under conditions in which synthesis of primer extension product is induced, i.e., in the presence of nucleotides and an agent for polymerisation such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerisation.

The exact lengths of the primers will depend on many factors, including temperature and composition (A/T en G/C content) of primer. A pair of bi-directional primers consists of one forward and one reverse primer as commonly used in the art of DNA amplification such as in PCR amplification.

It will be understood that "primer", as used herein, may refer to more than one primer, particularly in the case where there is some ambiguity in the information regarding the terminal sequence(s) of the target region to be amplified. Hence, a "primer" includes a collection of primer oligonucleotides containing sequences representing the possible variations in the sequence or includes nucleotides which allow a typical base pairing.

The oligonucleotide primers may be prepared by any suitable method. Methods for preparing oligonucleotides of specific sequence are known in the art, and include, for example, cloning and restriction of appropriate sequences, and direct chemical synthesis. Chemical synthesis methods may include, for example, the phospho di- or tri-ester method, the diethylphosphoramidate method and the solid support method disclosed in e.g. U.S. Pat. No. 4,458,066. The primers may be labelled, if desired, by incorporating means detectable by for instance spectroscopic, fluorescence, photochemical, biochemical, immunochemical, or chemical means.

Template-dependent extension of the oligonucleotide primer(s) is catalysed by a polymerising agent in the presence of adequate amounts of the four deoxyribonucleotide triphosphates (dATP, dGTP, dCTP and dTTP, i.e. dNTPs) or analogues, in a reaction medium which is comprised of the appropriate salts, metal cations, and pH buffering system. Suitable polymerizing agents are enzymes known to catalyse primer- and template-dependent DNA synthesis. Known DNA polymerases include, for example, E. coli DNA polymerase I or its Klenow fragment, T4 DNA polymerase, and Taq DNA polymerase. The reaction conditions for catalysing DNA synthesis with these DNA polymerases are known in the art.

The products of the synthesis are duplex molecules consisting of the template strands and the primer extension strands, which include the target sequence. These products, in turn, serve as template for another round of replication. In the second round of replication, the primer extension strand of the first cycle is annealed with its complementary primer; synthesis yields a "short" product which is bound on both the 5'- and the 3'-ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve.

The PCR method is well described in handbooks and known to the skilled person.

After amplification by PCR, the target polynucleotides may be detected by hybridisation with a probe polynucleotide which forms a stable hybrid with that of the target sequence under stringent to moderately stringent hybridisation and wash conditions. If it is expected that the probes will be essentially completely complementary (i.e., about 99% or greater) to the target sequence, stringent conditions will be used. If some mismatching is expected, for example if variant strains are expected with the result that the probe will not be completely complementary, the stringency of hybridisation may be lessened. However, conditions are chosen which rule out non-specific/adventitious binding. Conditions which affect hybridisation, and which select against non-specific binding are known in the art, and are described in, for example, Sambrook et al., (2001). Generally, lower salt concentration and higher temperature increase the stringency of binding. For example, it is usually considered that stringent conditions are incubations in solutions which contain approximately 0.1xSSC, 0.1% SDS, at about 65°C incubation/wash temperature, and moderately stringent conditions are incubations in solutions which contain approximately 1-2xSSC, 0.1% SDS and about 50°-65°C incubation/wash temperature. Low stringency conditions are 2xSSC and about 30°-50°C.

The terms "stringency" or "stringent hybridisation conditions" refer to hybridisation conditions that affect the stability of hybrids, e.g., temperature, salt concentration, pH, formamide concentration and the like. These conditions are empirically optimised to maximize specific binding and minimize non-specific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer. Typically, stringent conditions will be those in which the salt concentration is less than about 1.0 M $Na+$ ion, typically about 0.01 to 1.0 M $Na+$ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridisation with a buffer solution of 30% formamide, 1 M NaCl, 1% SDS at 37°C and a wash in 2x SSC at 40°C. Exemplary high stringency conditions include hybridisation in 50% formamide, 1 M NaCl, 1% SDS at 37°C, and a wash in 0.1x SSC at 60°C. Hybridisation procedures are well known in the art and are described by e.g. Ausubel et al., 1998 and Sambrook et al., 2001.

The term "fragmented genomic DNA" refers to pieces of DNA of the genome of a cell that are the result of the partial physical, chemical or biological break-up of the lengthy DNA into discrete fragments of shorter length.

The term "hybridization pattern" refers to the list of measurements of spot intensities obtained after hybridizing the array with a target nucleic acid.

The term "nucleotide" is used to denote a deoxyribonucleoside, a ribonucleoside, or a 2'-O-substituted ribonucleoside residue

The term "molecular marker" generally refers to markers identifying variation at the level of DNA and is herein used to refer to a mutation (of any type) or nucleotide sequence which has a scorable or selectable relation with either the pathogenic or non-pathogenic phenotype (and hence can "mark" a region of the chromosome).

It now has been found, from a series of experiments comparing pathogenic and non-pathogenic *Legionella* strains, that a good discrimination can be achieved by detecting the presence of at least two marker sequences. Said markers are indicated as the sequences of SEQ ID NO:1 and SEQ ID NO:2. In a typical experiment said markers are present on a hybridization array and DNA of a *Legionella* strain to be classified as pathogenic or non-pathogenic is allowed to hybridize with said markers.

In another embodiment of the present invention, the array comprises at least the five sequences as depicted in SEQ ID NO:1 through SEQ ID NO: 5. The sequences of the two and/or the five markers given in the sequence listing are about 1000 nucleotides long. However, this does not necessarily mean that the complete sequence of SEQ ID NO: 1-5 needs to be present on the array: the assay on pathogenicity can equally well been performed with sequences, which are parts of the SEQ ID NO:1-5. It should, however, be understood, that a minimal length is necessary for obtaining a correct and distinctive hybridization with any sample nucleic acid. Thus, the length of these part(s) of the sequences should range from about 50 to about 2000 nucleotides, i.e. the sequences can be shorter or longer than those presented in the sequence listing, but for a sufficient hybridization to occur they should be at least 50 nucleotides long. Longer sequences than those depicted can be obtained by hybridizing the genome from a *Legionella* species and, if hybridization occurs with the presented sequence, excise a larger part of the sequence from the genomic DNA...

Further, hybridization allows for some mismatches between the sequence on the array and the nucleotide sequence of the sample. Therefore, any nucleotide sequence or part thereof (as defined above) which has a homology of at least 70%, preferably at least 80%, more preferably at least 90%, more preferably at least 95%, even more preferably at least 99% with the sequences of SEQ ID NO:1 through SEQ ID NO:5 is applicable in and intended within the scope of the present invention.

In the current invention the term "homologues" is used to indicate both parts of the sequences of the invention of at least 50, but preferably at least 100, more preferably at least 200, more preferably at least 500 and most preferably at least 1000 nucleotides, and sequences with have a degree of homology of at least 70%, preferably at least 80%, more preferably at least 90%, more preferably at least 95%, even more preferably at least 99% with the sequences of SEQ ID NO:1 through SEQ ID NO:5.

The above discussed markers, or fragments thereof may be spotted on a surface to provide for a DNA micro-array. In order to facilitate coupling of the fragments, the surface of the array (e.g. the slide, the surface of which may i.a. be glass, gold, etc.) may be modified. Spotting may occur by any method available, for instance by using ElectroSpray Ionization (ESI) micro-array printing. After spotting of the markers or fragments thereof, the slide surfaces may be blocked to prevent further attachment of nucleic acids, e.g. by treatment with boro-anhydride in case of formaldehyde modified glass-slide surfaces.

To facilitate detection of successful hybridization, the gDNA is suitably labeled, preferably with a compound which is uniquely detectable by an antibody. Such a compound can for instance be biotin. Labeling of nucleotides with biotin and subsequent detection through antibodies specific for biotin, has been sufficiently described in the literature and will be routine experimentation for a person skilled in the art. Alternatively, the nucleotides are fluorescently labeled (e.g. by using CyTM labels [Amersham Pharmacia Biotech]). Fluorescent labeling kits are commercially available from various manufacturers. In order to be able to judge the signals caused by the hybridization of the marker sequences with sample nucleic acid, preferably the array also comprises reference nucleotide sequences, which can serve as positive and negative controls. As negative controls, sequences should be used of about the same length as the average length of the markers, but which will not hybridize with any nucleotide sequence in the sample, i.e. sequences, which do not occur in *Legionella.* As positive control, a sequence should be used which will be present in (nearly) all samples to be tested. For this purpose preferably the *Legionella* 30S ribosomal protein S21 (Cazalet,C., Rusniok,C., Bruggemann, H., Zidane,N., Magnier,A.,Ma,L., Tichit,M., Jarraud,S., Bouchier,C., Vandenesch,F., Kunst,F., Etienne,J., Glaser,P. and Buchrieser,C. Nat. Genet. 36 (11), 1165-1173 (2004)) or a conserved sequence of one of the housekeeping enzymes should be used.

The average size of sample nucleic acid has an effect on the signal distribution on the array. Larger sample molecules comprise more information and are thus more likely to find a suitable hybridization partner in more of the spots. Reducing the average size of the sample nucleic acid can reduce this phenomenon. On the other hand, when the sample nucleic acid is too small, the nucleic acid fragments in the sample contain too little genetic information and also find suitable hybridization partners in many spots. The average size of the fragments in the sample nucleic acid is preferably between about 30 and 3000 nucleotides. More preferably, the average size of the fragments in the sample nucleic acid comprises a size of between about 50 and 1000 nucleotides, more preferably between about 100 and 500 nucleotides.

In a method for detecting a pathogenic *L. pneumophila* strain, the sample nucleic acid may represent the whole or a part of the sample genome. Preferably, at least those parts of the genome are present in which the presence of molecular markers as defined herein is to be detected. This can be achieved by randomly digestion of the genomic DNA of the sample to fragments of about 1,5 kb or by physically fragmenting the DNA (e.g. by shearing) to form fragments of about that size. In a preferred embodiment a PCR amplification step is performed on either the intact genomic DNA or on the fragmented DNA with primers that specifically cause amplification of one or more of the sequences of the invention. Alternatively, the DNA can be randomly (primer) labeled using Klenow DNA polymerase (BioPrime kit Invitrogen) according to the manufacturer's instructions..

The fragmented sample DNA is then brought into contact with the array of the invention, which contains at least the two marker sequences of SEQ ID NO:1 and SEQ ID NO:2 or fragments thereof, or homologues of said markers or fragments thereof, or, in another embodiment, the five marker sequences of SEQ ID NO:1 through SEQ ID NO:5 or fragments thereof, or homologues of said markers or fragments thereof. Hybridization of the sample nucleotides with the marker sequences and the hybridization signals with optional positive and negative control sequences is then determined. Then, the diagnosis whether or not the sample contained a pathogenic *Legionella* strain is done according to the following logic rule for the assay with only SEQ ID NO: 1 and SEQ ID NO:2. In said rule the value for SEQ ID NO:1 or 2 is 1 if a positive hybridization signal is obtained with respect to said sequence, and it is 0 if no positive hybridization signal is detected.

If (SEQ ID NO:1 > SEQ ID NO:2) then Pathogenic else Environmental

For the assay with all five sequences, the following set of 7 rules is applicable. If 4 or more of these rules are TRUE if the stochastic 0 or 1 value for each hybridization is entered, then the sample can be classified as pathogenic.

If (SEQ ID NO:2 < SEQ ID NO:1) then [not(SEQ ID NO:2 = SEQ ID NO:3)] else [(SEQ ID NO:2 = SEQ ID NO: 4) and (SEQ ID NO:5 = SEQ ID NO:3)]

If (SEQ ID NO:2 < SEQ ID NO:3) then [not(SEQ ID NO:1 < SEQ ID NO:4)] else [(SEQ ID NO:5 > SEQ ID NO: 2) nor (SEQ ID NO:5 < SEQ ID NO:4)]

If (SEQ ID NO:2 < SEQ ID NO:3) then [not(SEQ ID NO:4 < SEQ ID NO:1)] else [(SEQ ID NO:3 < SEQ ID NO: 5) nor (SEQ ID NO:4 > SEQ ID NO:5)]

If (SEQ ID NO:1 = SEQ ID NO:5) then [(SEQ ID NO:4 <= SEQ ID NO:1)] else [(SEQ ID NO:1 < SEQ ID NO: 4) nor (SEQ ID NO:3 = SEQ ID NO:2)]

If (SEQ ID NO:1= SEQ ID NO:5) then [(SEQ ID NO:5 >= SEQ ID NO:4)] else [(SEQ ID NO:3 <= SEQ ID NO: 2) nor (not(SEQ ID NO:4 < SEQ ID NO:1))]

If (SEQ ID NO:1 = SEQ ID NO:5) then [not(SEQ ID NO:4 > SEQ ID NO:1)] else [(SEQ ID NO:4 > SEQ ID NO: 1) nor (SEQ ID NO:2 >= SEQ ID NO:3)]

If (SEQ ID NO:1 = SEQ ID NO:5) then [not(SEQ ID NO:1 < SEQ ID NO:4)] else [(SEQ ID NO:3 = SEQ ID NO: 2) nor (SEQ ID NO:1 < SEQ ID NO:4)]

Preferably, the assay kit of the invention comprises software, which is programmed to comprise the above logic rules, and which can - on basis of the stochastic values 0 or 1, resulting from the hybridization test - immediately yield the result of the test, i.e. whether the tested sample contains a pathogenic *Legionella* strain or not.

## EXAMPLES

### *DNA isolation of sample*

In the present Example a total of 144 samples from different strains were selected from the collection of strains of the Streeklaboratorium Kennemerland in Haarlem, The Netherlands. Of these 144 samples, a total of 74 samples was isolated from hospital patients (pathogenic or clinical strains) and a total of 70 samples was isolated from industrial and public water supply systems, and were not detected in humans (environmental strains).

### *Labeling and hybridisation of genomic DNA*

All strains were cultivated after which genomic DNA was isolated from these strains. The DNA was then randomly (primer) labeled using Klenow DNA polymerase (BioPrime kit Invitrogen) according to the manufacturer's instructions.

Cy3 labeled marker sequences were prepared. The DNA sample consisted of the DNA of the test strain and was labeled with Cy5. Both labeled samples were hybridized simultaneously to a microarray. Upon scanning and image analysis, the ratio of the Cy5/Cy3 emission values was calculated for each spot on the microarray. These ratio's served as data input for further data analysis.

All tests showed a positive control, i.e. the presence of *L. pneumophila* DNA was detected. Then, on basis of either positive or negative hybridization signals on the two markers (SEQ ID NO:1 and SEQ ID NO:2) or the five markers (SEQ ID NO: 1 through SEQ ID NO:5) a classification of the sample as 'pathogenic' or 'non-pathogenic', respectively, was generated according to the rules as shown above. For the results, the sensitivity and specificity of the tests were calculated.

Sensitivity is calculated as the number of true positive classifications divided by the sum of the number of true positives and the number of false negatives as expressed by equation 1:

$$\text{sensitivity} = TP/(TP=FN) \qquad (I)$$

wherein true positives are the number of pathogenic strains correctly identified and false negatives are the number of pathogenic strains incorrectly classified as being environmental strains.

Specificity is defined as the number of true negative results divided by the sum of the true negatives and the false positives as expressed by equation 2:

$$\text{specificity} = TN/(TN+FP) \qquad (II)$$

wherein true negatives are defined as the number of environmental strains that are correctly identified and false positives are defined as the number of environmental strains that are identified as being pathogenic.

| Array size | Sensitivity (%) | Specificity (%) |
|---|---|---|
| 5 markers | 95.8 | 61.4 |
| 2 markers | 86.3 | 68.6 |

SEQ ID NO: 1 (LePn.01gH4)

```
CTCGAGATCAGCTTGATTCGTTTTTATGTTTGATAAATGGTATGGCGAGGGGAGCCTATCACCCTGGTTTT
GGCATTAAATCGTTTTTTTGTAATTTCTGCAGCGTGATGATCTATGCATAAATAGCAATCCGCTAATTTTT
CAAAGAAAAAATTAACTTCTCCCCAAAAATCTTGAAGGATGAATGTTTTGATATGCGCGGGACACACAGCT
ATTATTGCTTCATCTATGCCAGCTTCGCCAGGAGATGATAATCCAGATAATATGATATCGGGATTTAATTT
ATCAATTAATTGATTGGCGTAGGATAGTAGATGATTGGATTCATTTGAGGATCGGGTTGGTGGTAGTGGAA
CTTGAAATACAGATTGAATCCCAGCCCGAAGAAAATATTTTGAAGCGGGAGGTTGAGCATAAATATATACA
GTAACACCTGTATATTGTTTAAGTATTTTTACTATCTCAATAATGTTTAATGCAGCACCAACATCCCGTGC
AGTAACAAGAACTCTCACACTATCTTTCTTATCCTTGTAAAATTGTTTTTATTAAATCCCGTTCATTTTCT
GCTTTCTGAGTTAATTTAAAAAATTTATCCCATTCCGAGTTCAGTATATTTTTATTTTTCTCTGCT
```

SEQ ID NO: 2 (LePn.011A2-b)

CNCGGAGATCAGCTNGATTCGCAGAGAATCCATTCGTTTATTTTTTTGAAGAATCGAGAATAAATTCCGGT
ATTTCAGGAACCATTCAAATGATTTTGTCGGGAAAAGATATGATGCCAGCTAGCGATTTTTCAAACGGGAT
TAGAGAGGAGGCCTATCTTTACTTAAAGCGCGGACCTCTTTCTTGGGATAAAAATCAAATTGATAAAGAGC
GTTTTTTAATTACGGATGTCTTAGAAGATATTTTATCTCCTAAGTCTTATGAAGAACAAATTGCCTCAGCC
TCATGGCTTTTTGAAGCATTGTCTCAATTTTATTTTAGAGCTCAAAATAAATGGTGCGCCAGTGGTAAATC
AATCATTCGCTATTTGCATCAAGAAAATCCCGATTTAGCCAGGGAGTTTTCTGAAAGTTTTAATCGAGTTT
ATAAGGAAGGTGATTCAACTCATTTAAAAAAGTTGGTTGAAAAAATACTACAACCTTATGGAGGTATTCTT
TGGAGCGGTTATCATTCCGATGCACCAGAAGATGCAAAAATACCAGAGCTTATAATTTTAGCCAAGAACAA
TGACTATGAGATTTGTTTCGAAGA

SEQ ID NO:3 (LePn.010B12)

CTCGGAGATCAGCTTGAATTCGTTTTTTCTGGGAATATGTCTTGGCATGCAGATGATGCTTTCCAAAAGTA
CTGAATTTGGACAACATGAAGGTCTGGGTCTAATTGCTGGCGAGGTTGTTAGCGTCCCTTCACATGGAGTT
GATGGTCAATTGCATAAAATACCTCATATAGGCTGGAATGAACTGGTTTCCACCTCGGAAGGCGAGGATTG
GTGTCATACTATCCTGAAAAATATACCATTAAATTCTTCAGTATATTTTGTTCATTCTTTTATGGCCATGC
CTAGTAATCCTAAAAAGCGTCTGGCTGATACTTTATATGATGGTCAGGCTATTAGCGCAGTAATAAAAGAT
GAAAATATGTACGGATGCCAATTTCATCCTGAAAAAAGCGGGGAAGTAGGTTTAAGCATCATTCAACAGTT
TTTGCAGATTTAGGGTGAATTAAAAATGAGAATATTGGCAGTAATCCCGGCAAGGGCTGGCTCAAAGAGGC
TGCCCGGTAAAAATACCAGATTACTTGCCGGAAAACCATTAATTGCACATACTATTGTTGCTGCCTTGCAG
TCGTCTTGTTGTGAAGA

SEQ ID NO:4 (LePn.024B1)

CTCGANATCAGCTTGAATTCGTTGGCATACACCGCCGCCCATGAGCCGTTCAAAAGCTGTAGTGAAGTATT
TACAAGAGCATATCAGTAAAGCCATCACTTCAGGCAACGTTGAAAGCCTTCATGATCAAGTGCTCATTGAT
CTGTTTGATGCGGCCATTATGGATAAAAATTATTACGATAAAATCACAGCCAGTGTTGGCCCTGTATTATC
AGAGATTAATAAGAGTAATGCCTCCAGGATCTTTTCATTTCATAAAAGCAATTGTGAAATCGAGTTGATGA
GTGCAATAAAAAACAAACAAGTCATCTATATCGGGCTGGATAGCTTAACAAACCCCAATATCGCTCAAGCA
GTCGGTAAAGCCTTTTTGTCAGATTTAGTATCCACTGCTGGAAAAATATATAAGGAAAGTAACGCGAATTA
CCGTCTCAATCTCCATTGCGATGAGCTTTCTGAAATTATCCAAGATTCGTTTGTTAAAATTTTAAATAAAG
CCGGTGGAGCTGGTTTCCAAGTCACAGCTTATGCTCAGACCAAACAAGATATGGAGGTAGCGCTAGGTTCA
AAAGCCAAAGCAGAAGTGACTGAAGGAAATCTAAACACCCTCATCATGTTACGTGTCAAAAATGAAGAAAC
TGCCAATTTATTAGTTAAAGTTTTACCAAAAATTGGCGTGGTCGAACATACTCAAGTCTCCATGGTCAAC

SEQ ID NO:5 (LePn.008D6)

CTCGGAGATCAGCTTGAATTCGTTTTCATTCATGCTGGATATACATATTCAGCAACATGGTTACCAGGAAA
TATATGTACCTTATATTGTTAATGCGGATAGCTTACTAGGGACTGGACAGTTACCAAAGTTTGAGGCCGAT
TTATTTAAATTAACAGGAGATAATGGGTACTACTTGACGTCTACTTCAGAAATTCCAGTGACCAATACAGT
AAGGGAAATGATATTGTCTGCTGAGCAATTACCTATTCGTTATGTTTGCCATTCCCCGTGTTTTCGTAGTG
AGGCGGGTTCTTACGGTAAAGACACAAAGGGAATGATTAGACAGCATCAATTTGAGAAAGTTGAACTGGTC
TGGATTACCAAACCGGAAGATTCTTATAATGCTTTAGAGCAACTGACTCAGCATGCTGAAGTTATTTTACA
GCGTTTGAACTTGCCTTACCGAGTGGTTGCTTTGTGTACCGGGGATATAGGCGCCGGTTCAGCCAAAACCT
ATGATCTTGAGGTTTGGTTGCCTAGCCNGAATACTTATCGAGAAATATCCTCTTGTTCCAATATGGAGGCA
TTTCAGGCACGCCGAATGAAAGCGCGTTTCCGCAATCCGGATACTAATGAGATTCAATTAGTCCACACTCT
AAATGGATCAGGTTTAGCCGTTGGAAGAACTTTAGTTGCCATTATGG

## Claims

1. A method for distinguishing between clinical or pathogenic *Legionella* strains and environmental or non-pathogenic *Legionella* strains, wherein said method involves an hybridization assay with at least the specific genetic markers as depicted in SEQ ID NO:1 and SEQ ID NO:2 or homologues thereof.

2. A method according to claim 1, wherein the hybridization assay further comprises hybridization with the specific genetic markers as depicted in SEQ ID NO:3, SEQ ID NO:4 and SEQ ID NO:5 or homologues thereof.

3. A method according to claim 1 or 2, wherein the markers are hybridized with genomic DNA of a sample, preferably fragmented genomic DNA.

4. A method according to any of the preceding claims, wherein the size of the specific genetic markers is from about 50 to about 2000 nucleotides, more preferably from about 500 to about 1800 nucleotides, most preferably from about 1000 to about 1500 nucleotides.

**5.** A method according to claim 1, wherein the diagnosis that a pathogenic *Legionella* is present in the sample is reached by the logic rule
If (SEQ ID NO:1 > SEQ ID NO:2) then Pathogenic else Environmental
wherein SEQ ID NO:1 and SEQ ID NO:2 are stochastic values representing 1 if hybridization and 0 if no hybridization has been observed with said sequences.

**6.** A method according to claim 2, wherein the diagnosis that a pathogenic *Legionella* is present in the sample is reached if 4 or more of the logic rules 1-7

1. If (SEQ ID NO:2 < SEQ ID NO:1) then [not(SEQ ID NO:2 = SEQ ID NO:3)] else [(SEQ ID NO:2 = SEQ ID NO:4) and (SEQ ID NO:5 = SEQ ID NO:3)]
2. If (SEQ ID NO:2 < SEQ ID NO:3) then [not(SEQ ID NO:1 < SEQ ID NO:4)] else [(SEQ ID NO:5 > SEQ ID NO:2) nor (SEQ ID NO:5 < SEQ ID NO:4)]
3. If (SEQ ID NO:2 < SEQ ID NO:3) then [not(SEQ ID NO:4 < SEQ ID NO:1)] else [(SEQ ID NO:3 < SEQ ID NO:5) nor (SEQ ID NO:4 > SEQ ID NO:5)]
4. If (SEQ ID NO:1 = SEQ ID NO:5) then [(SEQ ID NO:4 <= SEQ ID NO:1)] else [(SEQ ID NO:1 < SEQ ID NO:4) nor (SEQ ID NO:3 = SEQ ID NO:2)]
5. If (SEQ ID NO:1 = SEQ ID NO:5) then [(SEQ ID NO:5 >= SEQ ID NO:4)] else [(SEQ ID NO:3 <= SEQ ID NO:2) nor (not(SEQ ID NO:4 < SEQ ID NO:1))]
6. If (SEQ ID NO:1 = SEQ ID NO:5) then [not(SEQ ID NO:4 > SEQ ID NO:1)] else [(SEQ ID NO:4 > SEQ ID NO:1) nor (SEQ ID NO:2 >= SEQ ID NO:3)]
7. If (SEQ ID NO:1 = SEQ ID NO:5) then [not(SEQ ID NO:1 < SEQ ID NO:4)] else [(SEQ ID NO:3 = SEQ ID NO:2) nor (SEQ ID NO:1 < SEQ ID NO:4)],

wherein SEQ ID NO:n (n=1-5) is the stochastic value representing 1 if hybridization and 0 if no hybridization has been observed with said sequence n, have the result TRUE.

**7.** A hybridization assay kit which comprises less than 50 nucleotides and which comprises at least SEQ ID NO:1 and SEQ ID NO:2 or homologues thereof.

**8.** A hybridization assay kit according to claim 7, which further comprises SEQ ID NO:3, SEQ ID NO:4 and SEQ ID NO:5 or homologues thereof.

**9.** A hybridization assay kit according to claim 7 or 8, which further comprises a positive control sequence, preferably *Legionella* 30S ribosomal protein S21 or a conserved sequence of one of the *Legionella* housekeeping enzymes.

**10.** A hybridisation assay kit according to any of claims 7-9, which further comprises software for calculating the results of the logic rules represented in claim 5 or 6.

European Patent Office

# EUROPEAN SEARCH REPORT

Application Number
EP 06 07 7343

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 92/11273 A (HOFFMANN LA ROCHE [CH]) 9 July 1992 (1992-07-09) * the whole document * ----- | 1-10 | INV. C12Q1/68 |
| A | WO 2005/049642 A (PASTEUR INSTITUT [FR]; INST NAT SANTE RECH MED [FR]; UNIV CLAUDE BERNA) 2 June 2005 (2005-06-02) * abstract * ----- | 1-10 | |
| A | US 6 194 145 B1 (HEIDRICH BJOERN [DE] ET AL) 27 February 2001 (2001-02-27) * the whole document * ----- | 1-10 | |
| A | DATABASE GENBANK 19 June 2003 (2003-06-19), WANG L ET AL.: "Prokaryotic essential gene #18982" XP002439895 Database accession no. ACA37325 * abstract * | 1-10 | |
| A | & WO 02/077183 A (ELITRA PHARMACEUTICALS INC [US]; WANG LIANGSU [US]; ZAMUDIO CARLOS [US) 3 October 2002 (2002-10-03) * abstract * ----- | 1-10 | TECHNICAL FIELDS SEARCHED (IPC) C12Q |
| A | DATABASE GENBANK 19 June 2003 (2003-06-19), WANG L ET AL:: "Prokaryotic essential gene #18687" XP002439896 Database accession no. ACA37030 * abstract * | 1-10 | |
| A | & WO 02/077183 A (ELITRA PHARMACEUTICALS INC [US]; WANG LIANGSU [US]; ZAMUDIO CARLOS [US) 3 October 2002 (2002-10-03) * abstract * ----- -/-- | 1-10 | |

The present search report has been drawn up for all claims

| Place of search | Date of completion of the search | Examiner |
|---|---|---|
| Munich | 23 July 2007 | Florey, Marianne |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 07 7343

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE GENBANK 19 June 2003 (2003-06-19), WANG L ET AL.: "Prokaryotic essential gene #18798" XP002439897 Database accession no. ACA37141 * abstract * | 1-10 | |
| A | & WO 02/077183 A (ELITRA PHARMACEUTICALS INC [US]; WANG LIANGSU [US]; ZAMUDIO CARLOS [US] 3 October 2002 (2002-10-03) * abstract * | 1-10 | |
| A | GAIA VALERIA ET AL: "Consensus sequence-based scheme for epidemiological typing of clinical and environmental isolates of Legionella pneumophila." JOURNAL OF CLINICAL MICROBIOLOGY MAY 2005, vol. 43, no. 5, May 2005 (2005-05), pages 2047-2052, XP002439888 ISSN: 0095-1137 * abstract * | 1-10 | TECHNICAL FIELDS SEARCHED (IPC) |
| A | LOENS K ET AL: "Development of conventional and real-time NASBA for the detection of Legionella species in respiratory specimens." JOURNAL OF MICROBIOLOGICAL METHODS DEC 2006, vol. 67, no. 3, December 2006 (2006-12), pages 408-415, XP005718129 ISSN: 0167-7012 * abstract * | 1-10 | |
| | -/-- | | |

The present search report has been drawn up for all claims

3

| Place of search | Date of completion of the search | Examiner |
|---|---|---|
| Munich | 23 July 2007 | Florey, Marianne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 07 7343

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RIFFARD S ET AL: "Distribution of mip-related sequences in 39 species (48 serogroups) of Legionellaceae." EPIDEMIOLOGY AND INFECTION DEC 1996, vol. 117, no. 3, December 1996 (1996-12), pages 501-506, XP009085911 ISSN: 0950-2688 * abstract * ----- | 1-10 | |
| A | CHIEN MINCHEN ET AL: "The genomic sequence of the accidental pathogen Legionella pneumophila." SCIENCE (NEW YORK, N.Y.) 24 SEP 2004, vol. 305, no. 5692, 24 September 2004 (2004-09-24), pages 1966-1968, XP002313850 ISSN: 1095-9203 * abstract * ----- | 1-10 | |
| A | POURCEL C ET AL: "Characterization of a tandem repeat polymorphism in Legionella pneumophila and its use for genotyping." JOURNAL OF CLINICAL MICROBIOLOGY MAY 2003, vol. 41, no. 5, May 2003 (2003-05), pages 1819-1826, XP002439889 ISSN: 0095-1137 * abstract * ----- -/-- | 1-10 | TECHNICAL FIELDS SEARCHED (IPC) |

The present search report has been drawn up for all claims

3

| Place of search | Date of completion of the search | Examiner |
|---|---|---|
| Munich | 23 July 2007 | Florey, Marianne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 07 7343

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AUREL HELENA ET AL: "Clinical and environmental isolates of Legionella pneumophila serogroup 1 cannot be distinguished by sequence analysis of two surface protein genes and three housekeeping genes." APPLIED AND ENVIRONMENTAL MICROBIOLOGY JAN 2005, vol. 71, no. 1, January 2005 (2005-01), pages 282-289, XP002439891 ISSN: 0099-2240 * abstract * ----- | 1-10 | |
| | | | TECHNICAL FIELDS SEARCHED (IPC) |

The present search report has been drawn up for all claims

| Place of search | Date of completion of the search | Examiner |
|---|---|---|
| Munich | 23 July 2007 | Florey, Marianne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

## ANNEX TO THE EUROPEAN SEARCH REPORT ON EUROPEAN PATENT APPLICATION NO. EP 06 07 7343

This annex lists the patent family members relating to the patent documents cited in the above-mentioned European search report.
The members are as contained in the European Patent Office EDP file on
The European Patent Office is in no way liable for these particulars which are merely given for the purpose of information.

23-07-2007

| Patent document cited in search report | | Publication date | Patent family member(s) | | | Publication date |
|---|---|---|---|---|---|---|
| WO 9211273 | A | 09-07-1992 | NONE | | | |
| WO 2005049642 | A | 02-06-2005 | FR | 2862659 | A1 | 27-05-2005 |
| US 6194145 | B1 | 27-02-2001 | AT | 227345 | T | 15-11-2002 |
| | | | DE | 19515891 | A1 | 31-10-1996 |
| | | | EP | 0739988 | A1 | 30-10-1996 |
| | | | ES | 2186745 | T3 | 16-05-2003 |
| | | | JP | 9107998 | A | 28-04-1997 |
| WO 02077183 | A | 03-10-2002 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5948902 A
- US 4458066 A


### Non-patent literature cited in the description


- **CAZALET,C ; RUSNIOK,C ; BRUGGEMANN,H ; ZIDANE,N ; MAGNIER,A ; MA,L ; TICHIT,M ; JARRAUD,S ; BOUCHIER,C ; VANDENESCH,F.** *Nat. Genet.,* 2004, vol. 36 (11), 1165-1173

SEQUENCE LISTING

<110> Nederlandse Organisatie voor Toegepast-Natuurwetenschappelijk Onderzoek TNO

<120> Method of detecting pathogenic Legionella strains

<130> P73508EP00

<140> EP 06077343.9
<141> 2006-12-29

<160> 5

<170> PatentIn version 3.3

<210> 1
<211> 634
<212> DNA
<213> Legionella pneumophila

<400> 1
```
ctcgagatca gcttgattcg tttttatgtt tgataaatgg tatggcgagg ggagcctatc      60
accctggttt tggcattaaa tcgttttttt gtaatttctg cagcgtgatg atctatgcat     120
aaatagcaat ccgctaattt ttcaaagaaa aaattaactt ctccccaaaa atcttgaagg     180
atgaatgttt tgatatgcgc gggacacaca gctattattg cttcatctat gccagcttcg     240
ccaggagatg ataatccaga taatatgata tcgggattta atttatcaat taattgattg     300
gcgtaggata gtagatgatt ggattcattt gaggatcggg ttggtggtag tggaacttga     360
aatacagatt gaatcccagc ccgaagaaaa tattttgaag cgggaggttg agcataaata     420
tatacagtaa cacctgtata ttgtttaagt atttttacta tctcaataat gtttaatgca     480
gcaccaacat cccgtgcagt aacaagaact ctcacactat ctttcttatc cttgtaaaat     540
tgtttttatt aaatcccgtt cattttctgc tttctgagtt aatttaaaaa atttatccca     600
ttccgagttc agtatatttt tatttttctc tgct                                 634
```

<210> 2
<211> 592
<212> DNA
<213> Legionella pneumophila

<220>
<221> misc_feature
<222> (2)..(2)
<223> n is a, c, g, or t

<220>
<221> misc_feature
<222> (15)..(15)
<223> n is a, c, g, or t

<400> 2
```
cncggagatc agctngattc gcagagaatc cattcgttta ttttttttgaa gaatcgagaa      60
taaattccgg tatttcagga accattcaaa tgattttgtc gggaaaagat atgatgccag     120
ctagcgattt ttcaaacggg attagagagg aggcctatct ttacttaaag cgcggacctc     180
```

tttcttggga taaaaatcaa attgataaag agcgtttttt aattacggat gtcttagaag 240

atattttatc tcctaagtct tatgaagaac aaattgcctc agcctcatgg ctttttgaag 300

cattgtctca attttatttt agagctcaaa ataaatggtg cgccagtggt aaatcaatca 360

ttcgctattt gcatcaagaa aatcccgatt tagccaggga gttttctgaa agttttaatc 420

gagtttataa ggaaggtgat tcaactcatt taaaaaagtt ggttgaaaaa atactacaac 480

cttatggagg tattctttgg agcggttatc attccgatgc accagaagat gcaaaaatac 540

cagagcttat aattttagcc aagaacaatg actatgagat ttgtttcgaa ga 592

<210> 3
<211> 585
<212> DNA
<213> Legionella pneumophila

<400> 3
ctcggagatc agcttgaatt cgtttttttct gggaatatgt cttggcatgc agatgatgct 60

ttccaaaagt actgaatttg gacaacatga aggtctgggt ctaattgctg gcgaggttgt 120

tagcgtccct tcacatggag ttgatggtca attgcataaa atacctcata taggctggaa 180

tgaactggtt tccacctcgg aaggcgagga ttggtgtcat actatcctga aaaatatacc 240

attaaattct tcagtatatt ttgttcattc ttttatggcc atgcctagta atcctaaaaa 300

gcgtctggct gatactttat atgatggtca ggctattagc gcagtaataa aagatgaaaa 360

tatgtacgga tgccaatttc atcctgaaaa aagcggggaa gtaggtttaa gcatcattca 420

acagtttttg cagatttagg gtgaattaaa aatgagaata ttggcagtaa tcccggcaag 480

ggctggctca aagaggctgc ccggtaaaaa taccagatta cttgccggaa aaccattaat 540

tgcacatact attgttgctg ccttgcagtc gtcttgttgt gaaga 585

<210> 4
<211> 709
<212> DNA
<213> Legionella pneumophila

<220>
<221> misc_feature
<222> (6)..(6)
<223> n is a, c, g, or t

<400> 4
ctcganatca gcttgaattc gttggcatac accgccgccc atgagccgtt caaaagctgt 60

agtgaagtat ttacaagagc atatcagtaa agccatcact tcaggcaacg ttgaaagcct 120

tcatgatcaa gtgctcattg atctgtttga tgcggccatt atggataaaa attattacga 180

taaaatcaca gccagtgttg gccctgtatt atcagagatt aataagagta atgcctccag 240

gatcttttca tttcataaaa gcaattgtga aatcgagttg atgagtgcaa taaaaaacaa 300

acaagtcatc tatatcgggc tggatagctt aacaaacccc aatatcgctc aagcagtcgg 360

taaagccttt ttgtcagatt tagtatccac tgctggaaaa atatataagg aaagtaacgc 420

gaattaccgt ctcaatctcc attgcgatga gctttctgaa attatccaag attcgtttgt 480
taaaatttta aataaagccg gtggagctgg tttccaagtc acagcttatg ctcagaccaa 540
acaagatatg gaggtagcgc taggttcaaa agccaaagca gaagtgactg aaggaaatct 600
aaacaccctc atcatgttac gtgtcaaaaa tgaagaaact gccaatttat tagttaaagt 660
tttaccaaaa attggcgtgg tcgaacatac tcaagtctcc atggtcaac 709

<210> 5
<211> 686
<212> DNA
<213> Legionella pneumophila

<220>
<221> misc_feature
<222> (525)..(525)
<223> n is a, c, g, or t

<400> 5
ctcggagatc agcttgaatt cgttttcatt catgctggat atacatattc agcaacatgg 60
ttaccaggaa atatatgtac cttatattgt taatgcggat agcttactag ggactggaca 120
gttaccaaag tttgaggccg atttatttaa attaacagga gataatgggt actacttgac 180
gtctacttca gaaattccag tgaccaatac agtaagggaa atgatattgt ctgctgagca 240
attacctatt cgttatgttt gccattcccc gtgttttcgt agtgaggcgg gttcttacgg 300
taaagacaca aagggaatga ttagacagca tcaatttgag aaagttgaac tggtctggat 360
taccaaaccg gaagattctt ataatgcttt agagcaactg actcagcatg ctgaagttat 420
tttacagcgt ttgaacttgc cttaccgagt ggttgctttg tgtaccgggg atataggcgc 480
cggttcagcc aaaacctatg atcttgaggt ttggttgcct agccngaata cttatcgaga 540
aatatcctct tgttccaata tggaggcatt tcaggcacgc cgaatgaaag cgcgtttccg 600
caatccggat actaatgaga ttcaattagt ccacactcta aatggatcag gtttagccgt 660
tggaagaact ttagttgcca ttatgg 686